# EUROPEAN PATENT APPLICATION

(11) **EP 2 431 057 A2**
(43) Date of publication of application: **21.03.2012**
(21) Application number: 10775048.1
(22) Date of filing: 20.04.2010
(51) Int. Cl.: A61L 2/07, A61L 2/18, B05B 7/08, B05B 9/04, B05B 12/10, B05B 1/24, B05B 1/30

(54) **STEAM STERILIZATION APPARATUS**

(30) Priority: 11.05.2009 KR 20090040929; 11.05.2009 KR 20090040930; 11.05.2009 KR 20090040933
(71) Applicant: Eco Inntot Co., Ltd., Ulsan 681-802 (KR)
(72) Inventor: LEE, Jong Chul, Ulsan 680-808 (KR)
(74) Representative: Dr. Weitzel & Partner
(86) International application number: PCT/KR2010/002452
(87) International publication number: WO 2010/131846

(57) **Abstract**

A steam sterilization apparatus, comprising:
a case forming a main body, a water container arranged in an inner rear portion of the case to store water;
a liquid chemical container arranged behind the water container to store a liquid chemical;
a nozzle assembly which is arranged in an inner front portion of the case and which includes a steam nozzle for receiving water from the water container and spraying water to the outside and a liquid chemical nozzle for receiving a liquid chemical from the liquid chemical container and discharging the liquid chemical to the outside;
a water supply pipe which interconnects the water container and the steam nozzle;
a liquid chemical supply pipe which interconnects the liquid chemical container and the liquid chemical nozzle;
a heater block interposed between the water supply pipe and the steam nozzle to heat and vaporize the water supplied through the water supply pipe;
a pump installed at the water supply pipe to apply pressure to the water in the water container and to supply water to the heater block through the water supply pipe; and
a control unit which controls the operation of the pump and of the heater block.

## Description

### [Technical Field]

The present invention relates to a steam sterilization apparatus, and more particularly, to a steam sterilization apparatus in which a crack in a vaporization space is minimized to achieve maximized water vaporization efficiency and to reduce the size of the apparatus, and which prevents liquid phase water from being sprayed at an early stage of operation of the apparatus to achieve a steam sterilization effect from the early stage of operation of the apparatus.

### [Background Art]

In general, a steam sterilization apparatus represents an apparatus that achieves a sterilization effect by mixing liquid chemical with sprayed steam and a representative steam sterilization apparatus which is known in the related art will be described below.

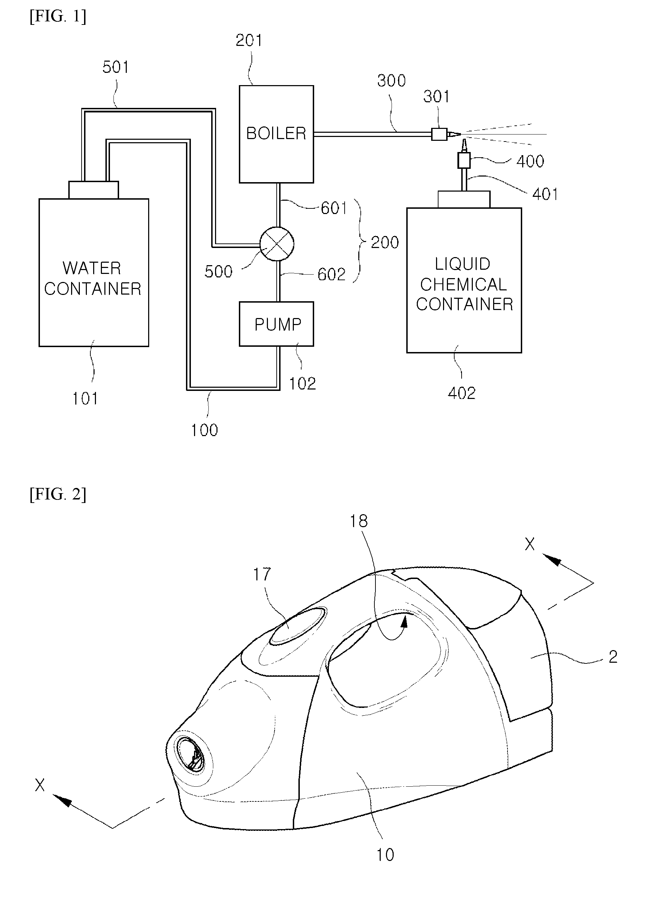

FIG. 1 is an overall configuration diagram showing "Spray Apparatus (hereinafter, referred to as a "steam sterilization apparatus in the related art")" of Korean Patent Registration No. 712639.

Referring to FIG. 1, the steam sterilization apparatus in the related art includes a water container 101 storing water therein, a pump 102 drawing out water from the water container 101, a boiler 201 heating the water drawn out from the pump 102 to convert the heated water into steam, a steam spray nozzle 301 for spraying the steam acquired through the boiler 201 to the outside, a liquid chemical container 402 storing liquid chemical therein, and a liquid chemical nozzle 400 for discharging liquid chemical from the liquid chemical container 402.

Further, the water container 101 and the pump 102 are connected to each other by a first water supply pipe 100, the pump 102 and the boiler 201 are connected to each other by a second water supply pipe 200, and the boiler 201 and the steam spray nozzle 301 are connected to each other by a steam pipe 300, and the liquid chemical nozzle 400 is connected with the liquid chemical container 402 by a liquid chemical supply pipe 401, and a direction changing valve 500 is installed at the second water supply pipe 200, such that the second water supply pipe 200 is divided into a discharge pipe 602 and a boiler water supply pipe 601 that are connected to the pump 102 around the direction changing valve 500 and the direction changing valve 500 and the water container 101 are connected to each other by a return pipe 501.

In addition, the steam spray nozzle 301 and the liquid chemical nozzle 400 are placed close to each other while forming an angle of 90° with each other, such that as steam is sprayed through the steam spray nozzle 301 at high pressure, pressure in the vicinity of the steam spray nozzle 301 is sharply decreased, and as a result, the steam and the liquid chemical are mixed with each other to be sprayed in a gaseous state while the liquid chemical is discharged to the outside through the liquid chemical nozzle 400 in a Ventura method.

However, in the case of using the steam sterilization apparatus in the relate art, problems to be described below occur.

First, the boiler 201 is not heated at an initial stage of operation of the steam sterilization apparatus not to heat water that flows into the boiler 201 to be discharged through the steam spray nozzle 301 in a liquid phase, and as a result, a problem in which a sterilization effect through the steam in the initial stage of operation cannot be achieved occurs.

Second, het generated from the boiler 201 while using the steam sterilization apparatus is rapidly transferred to the pump through the second water supply pipe 200 made of a metallic material, such that the pump 102 is easy to be damaged due to the transfer heat and a pulsation phenomenon occurs in water moved as the pump 102 operates, such that the steam is irregularly sprayed.

Third, a space provided in the boiler 201 to heat the water that flows in from the water container, i.e., a vaporization passage is constituted by one heat wire pole, such that generation efficiency of the steam is deteriorated and the boiler 201 having the structure has a large volume to cause the entire volume of the steam sterilization apparatus to be increased, such that mobility of the steam sterilization apparatus is deteriorated.

Fourth, since the liquid chemical container for storing the liquid chemical is provided in the steam sterilization apparatus as a fixed type, all the liquid chemical should be discharged by turning over a body of the sterilization apparatus in order to replace or clean the liquid chemical and when existing liquid chemical is not completely discharged, liquid chemical to be replaced is mixed with liquid chemical that remains in the liquid chemical container are mixed with each other to generate a toxic material.

### [Disclosure]

### [Technical Problem]

Therefore, in order to solve all the problems described above, an object of the present invention is to provide a steam sterilization apparatus capable of preventing liquid phase water from being sprayed at an initial stage of operation by preheating a heater block in reoperation by collecting water remaining in the heater block to a water container after operation and allowing the water to flow into the heater block.

Further, an object of the present invention is to provide a steam sterilization apparatus capable of achieving maximized vaporization efficiency and reducing the size of the steam sterilization apparatus by forming a multi-stage vaporization space in which a crack in a heater block is minimized.

In addition, an object of the present invention is to provide a steam sterilization apparatus capable of easily replacing and cleaning liquid chemical by removably configuring a liquid chemical container mounted in the steam sterilization apparatus.

### [Technical Solution]

An steam sterilization apparatus according to the present invention includes: a case forming a main body, a water container arranged in an inner rear portion of the case to store water; a liquid chemical container arranged behind the water container to store a liquid chemical; a nozzle assembly which is arranged in an inner front portion of the case and which includes a steam nozzle for receiving water from the water container and spraying water to the outside and a liquid chemical nozzle for receiving a liquid chemical from the liquid chemical container and discharging the liquid chemical to the outside; a water supply pipe which interconnects the water container and the steam nozzle; a liquid chemical supply pipe which interconnects the liquid chemical container and the liquid chemical nozzle; a heater block interposed between the water supply pipe and the steam nozzle to heat and vaporize the water supplied through the water supply pipe; a pump installed at the water supply pipe to apply pressure to the water in the water container and to supply water to the heater block through the water supply pipe; and a control unit which controls the operation of the pump and of the heater block.

### [Advantageous Effects]

In a steam sterilization apparatus according to the present invention, by recollecting water remaining in a heater block to a water container while stopping a pump and a heater block after operation, water is prevented from being sprayed as a liquid phase in reoperation to achieve a sterilization effect through steam from an initial stage of operation and the steam can be sprayed constantly by preventing a pulsation phenomenon from occurring in water moved through a water pressure balancing unit capable of constantly maintaining water pressure in operation.

Further, in the steam sterilization apparatus according to the present invention, the steam can be continuously sprayed with a constant discharge rate as evaporation efficiency of passing water is maximized by minimizing a crack of a vaporization space formed in the heater block and the size of the steam sterilization apparatus can be reduced by decreasing the volume of the heater block, such that it is easy to carry and move the steam sterilization apparatus.

In addition, in the steam sterilization apparatus according to the present invention, by configuring a liquid chemical container storing liquid chemical to be attached to or detached from a case of the steam sterilization apparatus, it is easy to replace and clean the liquid chemical.

### [Description of Drawings]

FIG. 1 is a block diagram of a configuration of a steam sterilization apparatus in the related art.
FIG. 2 is a perspective view of an external configuration of a steam sterilization apparatus according to the first exemplary embodiment of the present invention.
FIG. 3 is a cross-sectional view showing an internal configuration of a steam sterilization apparatus taken along line x-x shown in FIG. 2.
FIG. 4 is a perspective view of a heater block configuring the steam sterilization apparatus according to the first exemplary embodiment of the present invention.
FIG. 5 is a longitudinal cross-sectional view of a heater block taken along line A-A shown in FIG. 4.
FIG. 6 is a transverse cross-sectional view of a heater block taken along line B-B shown in FIG. 4.
FIG. 7 is an enlarged diagram of part C shown in FIG. 6.
FIG. 8 is a bottom view of the steam sterilization apparatus according to the first exemplary embodiment of the present invention.
FIG. 9 is a diagram showing an internal configuration of a steam sterilization apparatus according to a second exemplary embodiment of the present invention.
FIG. 10 is a side view specifically showing a configuration of main parts of the steam sterilization apparatus according to the second exemplary embodiment of the present invention.
FIG. 11 is a cross-sectional view of a water pressure balancing unit shown in FIG. 10.
FIG. 12 is an exploded perspective view of the water pressure balancing unit shown in FIG. 10.

### [Best Mode]

Hereinafter, exemplary embodiments of the present invention will be described below with reference to the accompanying drawings.

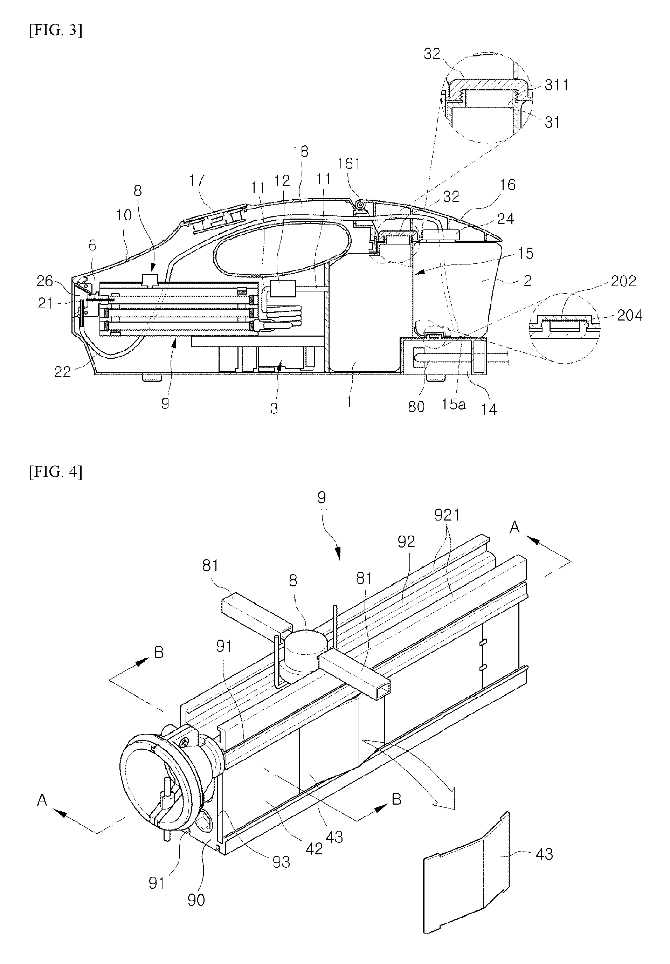

FIG. 2 is a perspective view of an external configuration of a steam sterilization apparatus according to the first exemplary embodiment of the present invention and FIG. 3 is a cross-sectional view showing an internal configuration of a steam sterilization apparatus taken along line x-x shown in FIG. 2.

Referring to FIGS. 2 and 3, the steam sterilization apparatus according to the first exemplary embodiment of the present invention includes a case 10 forming a main body, a water container 1 arranged in an inner rear portion of the case 10 to store water, a liquid chemical container 2 arranged behind the water container 1 to store a liquid chemical; a nozzle assembly 26 which is arranged in an inner front portion of the case 10 and which includes a steam nozzle 6 for receiving water from the water container and spraying water to the outside and a liquid chemical nozzle 21 for receiving a liquid chemical from the liquid chemical container 2 and discharging the liquid chemical to the outside; a water supply pipe 11 which interconnects the water container 1 and the steam nozzle 6, a liquid chemical supply pipe 22 which interconnects the liquid chemical container 2 and the liquid chemical nozzle 21; a heater block 9 interposed between the water supply pipe 11 and the steam nozzle 6 to heat and vaporize the water supplied through the water supply pipe 11; a pump 12 installed at the water supply pipe 11 to apply pressure to the water in the water container 1 and to supply water to the heater block 9 through the water supply pipe 11; and a control unit 3 which controls the operation of the pump 12 and of the heater block 9.

The case 10 forms an outer appearance of the steam sterilization apparatus and includes the nozzle assembly 26, the heater block 9, the pump 12, the control unit 3, the water container 1, and the liquid chemical container 2. Further, an operating unit 17 for controlling the operation of the control unit 3 is installed in an upper front portion of the case 10 and a handle unit 18 of which both sides are opened is installed in an upper middle portion to achieve use convenience.

In addition, a liquid chemical container receiving unit 15 for mounting the liquid chemical container 2 is installed in the inner rear portion of the case 10 and a lower portion of the case 10 where the liquid chemical container receiving unit 15 is installed is opened and includes a cable mounting space 14 capable of receiving a power cable 80.

A liquid chemical container receiving space 15a for mounting the liquid chemical container 2 is formed in the liquid chemical container receiving unit 15 and an upper cover unit 16 opened and closed through a front hinge portion 161 is formed in an upper portion of the liquid chemical container receiving space 15a to supply water to the water container 1 and attach or detach the liquid chemical container 2. Herein, the liquid chemical container receiving space 15a is opened to expose at least one side of the liquid chemical container 2 to the outside to check the amount of a liquid chemical remaining in the liquid chemical container 2 during using the steam sterilization apparatus.

The steam nozzle 6 constituting the nozzle assembly 26 is connected to the heater block 9 at an inlet thereof to spray water heated at a high temperature in the heater block 9 in a steam state and the liquid chemical nozzle 21 is connected to the liquid chemical supply pipe 22 at an inlet thereof and connected to the liquid chemical container 2 to discharge the liquid chemical to the outside. In this case, an outlet of the liquid chemical nozzle 21 is placed close to an outlet of the steam nozzle 6 to be perpendicular to each other, such that as steam is sprayed through the outlet of the seam nozzle 6 at a high pressure, a pressure in the vicinity of the liquid chemical nozzle 21 is rapidly decreased to discharge the liquid chemical to the outside through the outlet of the liquid chemical nozzle 21 in a Ventura method.

The heater block 9 includes a main body 90 with a vaporization passage 5 vaporizing water that flows thereinto while moving the water to an upper portion and a pair of heater plates 42 installed at both sides of the body 90 to vaporize the water in the vaporization passage 5 by heating the body 90. The heater block 9 heats the water that flows thereinto from the water container 1 through the water supply pipe 11 to the steam state and a detailed configuration of the heater block 9 will be described in detail through descriptions of FIGS. 4 to 7.

Further, the pump 12 is installed in the water supply pipe 11 and applies a pressure to the water supply pipe 11 by operating according to a control signal transmitted from the control unit 3 to enable the water moving in the water supply pipe 11 to flow into the heater block 9.

Hereinafter, the detailed configuration of the heater block which is the characteristic configuration of the steal sterilization apparatus according to the first exemplary embodiment of the present invention and a mounting structure of the liquid chemical container will be described in more detail.

### [Heater Block]

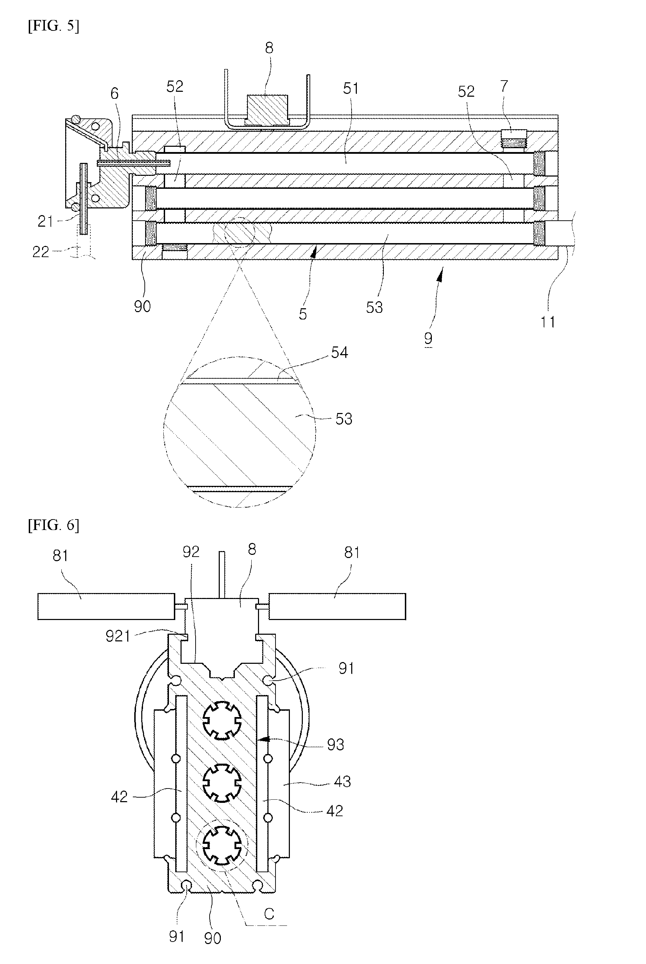

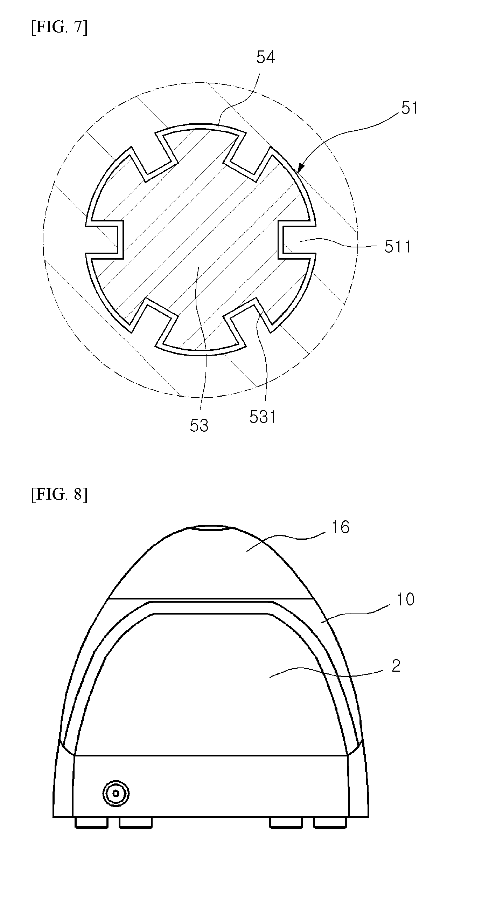

FIG. 4 is a perspective view of a heater block configuring the steam sterilization apparatus according to the first exemplary embodiment of the present invention, FIG. 5 is a longitudinal cross-sectional view of a heater block taken along line A-A shown in FIG. 4, FIG. 6 is a transverse cross-sectional view of a heater block taken along line B-B shown in FIG. 4, and FIG. 7 is an enlarged diagram of part C shown in FIG. 6.

Referring to FIGS. 4 to 6, the heater block 9 includes the body 90 which has heater plate insertion grooves 93 formed on both sides thereof to be buried with a predetermined depth and the vaporization passage 5 receiving water through the water supply pipe 11, which is formed therein, and a pair of heater plates 42 inserted into the heater plate insertion grooves 93 formed on both sides of the body 90 to vaporize the water that flows into the vaporization passage 5 by heating the water. Further, a pair of spring plates 43 are inserted into the pair of heater plate insertion grooves 93, respectively to strongly fix the heater plates 42 by pressing outer portions of the heater plates 42 inserted into the heater plate insertion grooves 93.

In addition, the vaporization passage 5 formed in the body 90 includes multi-stage horizontal passages 51 in which a rear portion of a lowermost end is opened and a front portion of an uppermost end is opened and a plurality of vertical communication holes 52 interconnecting the multi-stage horizontal passages 51 in a vertical direction.

The water supply pipe 11 is connected to an opened portion of the horizontal passage 51 positioned at the lowermost end and the steam nozzle 6 is connected to an opened portion of the horizontal passage 51 positioned at the uppermost end among the multi-stage horizontal passages 51.

Further, in each horizontal passage 51, a center shaft 53 is provided at the center in a longitudinal direction with a predetermined distance from an inner diameter of the horizontal passage 51 and a vaporization space 54 where water is vaporized on an outer periphery of the center shaft 53 and an inner periphery of the horizontal passage 51 is formed, as shown in FIGS. 5 and 7. In this case, a plurality of protruded portions 511 and buried portions 531 are formed in the horizontal passage 51 and the center shaft 53 in the longitudinal direction to increase a vaporized area of water. By this configuration, the size of the steam sterilization apparatus can be minimizing by reducing the volume of the heater block 9 while vaporization efficiency of water can be maximized by minimizing a crack in the vaporization passage 54, and the amount of steam sprayed to the outside can be sufficiently ensured, such that steam can be sprayed without cut-off of the steam.

In the body 90 of the heater block 9, a steam temperature sensor 7 provided in an upper portion of one of the vertical communication holes 52 to sense the temperature of steam in the vaporization passage 5 and output the sensed temperature to the control unit 3 may be additionally installed.

In addition, an upper buried groove 92 including both projection 921 formed longitudinally at front and rear portions is further provided in the upper portion of the body 90 of the heater block 9 as shown in FIG. 4 and a block temperature sensor 8 which senses the total temperature of the heater block 9 and outputs the sensed temperature to the control unit 3 is mounted on the buried groove 92.

The control unit 3 appropriately controls the operation of the heater plate 42 by using information outputted through the steam temperature sensor 7 and the block temperature sensor 8 to generate steam by effectively heating the water moving in the vaporization passage 5, and prevent power from being unnecessarily lost while preventing the heater block 9 from being excessively overheated and damaged.

Further, horizontal fixing bars 81 for fixing the heater block 9 to the case 10 of the steam sterilization apparatus are installed at both sides of the block temperature sensor 8 in the heater block 9 and a plurality of fixing grooves 91 for fixing the heater block 9 to the case 10 of the steam sterilization apparatus are installed at both upper sides and a bottom of the body 90 of the heater block 9.

### [Liquid Chemical Container]

FIG. 8 is a bottom view of the steam sterilization apparatus according to the first exemplary embodiment of the present invention and is described in association with FIG. 3.

In the steam sterilization apparatus according to the first exemplary embodiment of the present invention, the water container 1 received therein is fixedly mounted on the inside of the case 10 and the liquid chemical container 2 storing the liquid chemical is mounted on the inside of the case 10 to be attachable or detachable as described above.

Water such as ion water is stored in the water container 1 and a protruded pipe 31 opened eccentrically in an upper rear direction, i.e., a direction in which the liquid chemical container 2 is mounted is formed in the water container 1. A helix portion 311 is formed in an outer diameter of the protruded pipe 31.

Further, a press plug 32 joined to the helix portion 311 of the protruded pipe 31 to close the protruded pipe 31 is coupled to the water container 1. In this case, one portion of the press plug 32 joined to the helix portion 311, i.e., a portion contacting the water container 1 is broad to press an upper portion of the liquid chemical container 2 mounted in the rear portion of the water container 1, thereby preventing the liquid chemical container 2 mounted on the liquid chemical container receiving unit 15 from being removed.

An inlet (not shown) having a predetermined size is formed so as to inject the liquid chemical into the liquid chemical container 2 and an upper plug 24 is coupled to the inlet to be closed. Further, a buried groove 202 is formed on a bottom of the liquid chemical container 2 to be inserted and coupled to a protruded portion 204 formed in the liquid chemical container receiving unit 15, such that the liquid chemical container 2 is fixed to the inside of the case 10. In this case, groove portions having predetermined heights are formed on an outer peripheral surface of the protruded portion 204 and an inner peripheral surface of the buried groove 202, such that the liquid chemical container 2 can be more strongly fixed to the inside of the case 10.

Further, since the liquid chemical container 2 is mounted in the liquid chemical container receiving unit 15 with at least the rear portion of the liquid chemical container 2 exposed to the outside as shown in FIG. 8, the amount of the liquid chemical remaining in the liquid chemical container 2 during using the steam sterilization apparatus can be checked in real time, and as a result, the liquid chemical can be quickly supplemented depending on the amount of the remaining liquid chemical.

An operational relationship of the steam sterilization apparatus according to the first exemplary embodiment of the present invention configured as above will be described below.

First, when the apparatus is operated by user' operation, the control unit 3 operates the heater plate 42 to increase the temperature of the body 90 of the heater block 9 according to an operation signal and operates the pump 12 provided in the water supply pipe 11 to allow the water stored in the water container 1 to flow into the vaporization passage 5 formed in the body 90 of the heater block 9 through the water supply pipe 11.

The water that flows into the vaporization passage 5 in the body 90 flows into the vaporization space 54 of the horizontal passage 51 positioned on the lowermost end to be vaporized and moves to the horizontal passage 51 positioned on the uppermost end through the vertical communication hole 52 that interconnects the horizontal passages 51 in the vertical direction as the water is vaporized to be sprayed to the outside through the steam nozzle 6 connected to a front end of the horizontal passage 51 at the high pressure by an internal pressure of the vaporization passage 54.

In this case, while the steam is sprayed to the outside through the steam nozzle 6 at the high pressure, the pressure in the vicinity of the steam nozzle 6 is quickly decreased, and as a result, the liquid chemical is discharged from the liquid chemical nozzle 21 vertically arranged and fixed to the steam nozzle 6 in the Ventura method and mixed with the steam to be sprayed to the outside.

When the liquid chemical is short or needs to be replaced during using the steam sterilization apparatus, the steam sterilization apparatus is stopped by using the operating unit 17 and the liquid chemical container 2 is removed from the case 10 by opening the upper cover unit 16, and thereafter, the liquid chemical container 2 may be supplemented with the liquid chemical or the present liquid chemical may be replaced with other types of liquid chemicals.

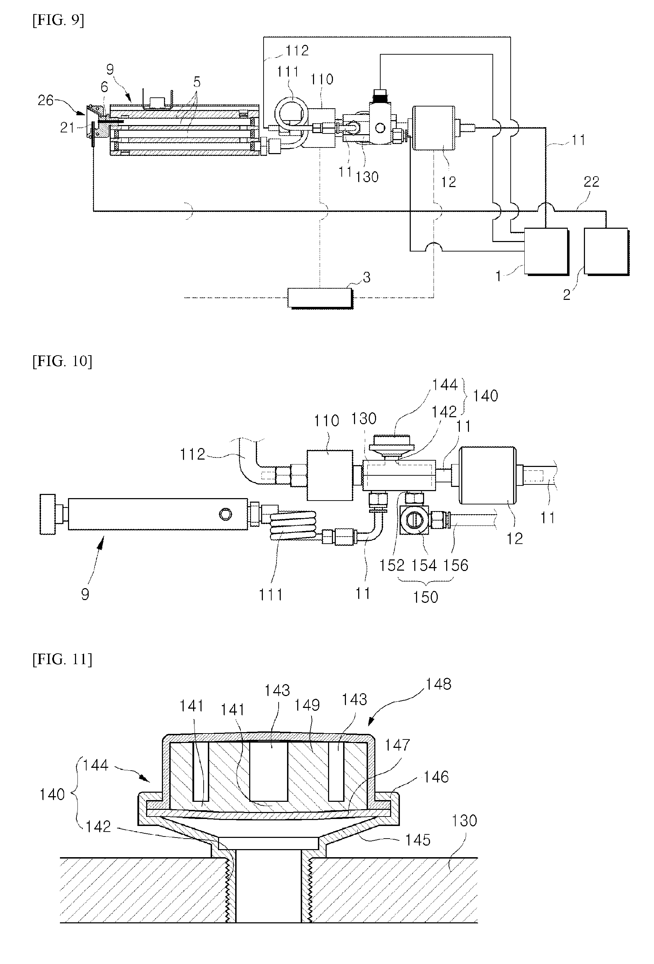

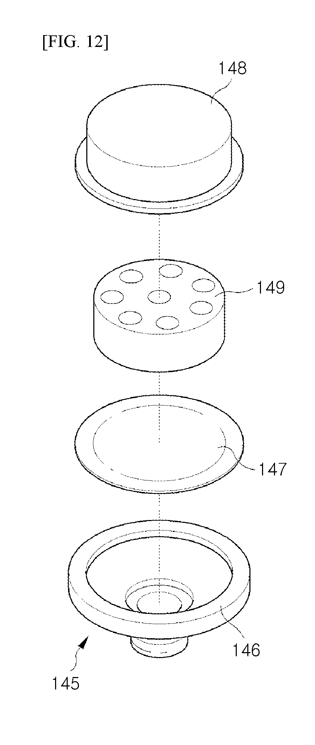

FIG. 9 is a diagram showing an internal configuration of a steam sterilization apparatus according to a second exemplary embodiment of the present invention, FIG. 10 is a side view specifically showing a configuration of main parts of the steam sterilization apparatus according to the second exemplary embodiment of the present invention, FIG. 11 is a cross-sectional view of a water pressure balancing unit shown in FIG. 10, and FIG. 12 is an exploded perspective view of the water pressure balancing unit shown in FIG. 10.

The steam sterilization apparatus according to the second exemplary embodiment of the present invention includes a case 10 forming a main body, a water container 1 arranged in an inner rear portion of the case 10 to store water, a liquid chemical container 2 arranged behind the water container 1 to store a liquid chemical; a nozzle assembly 26 which is arranged in an inner front portion of the case 10 and which includes a steam nozzle 6 for receiving water from the water container 1 and spraying water to the outside and a liquid chemical nozzle 21 for receiving a liquid chemical from the liquid chemical container 2 and discharging the liquid chemical to the outside; a water supply pipe 11 which interconnects the water container 1 and the steam nozzle 6, a liquid chemical supply pipe 22 which interconnects the liquid chemical container 2 and the liquid chemical nozzle 21; a heater block 9 interposed between the water supply pipe 11 and the steam nozzle 6 to heat and vaporize the water supplied through the water supply pipe 11; a pump 12 installed at the water supply pipe 11 to apply pressure to the water in the water container 1 and to supply water to the heater block 9 through the water supply pipe 11; and a control unit 3 which controls the operation of the pump 12 and of the heater block 9, and the water supply pipe 11 further includes a solenoid valve 110 connected to the water container 1 through a drain pipe 112 in order to recollect the water remaining in the heater block 9 to the water container 1 after the operation of the steam sterilization apparatus is terminated. Further, a heat dissipating pipe 111 for preventing heat generated from the heater block 9 from being transferred to the water supply pipe 11 may be additionally provided at a connection portion between the water supply pipe 11 and the heater block 9.

The exemplary embodiment has the same configuration as the first exemplary embodiment except for the configuration of the solenoid valve 110 connected to the water supply pipe 2.

Further, in the exemplary embodiment, a phenomenon in which the water remaining in the heater block 9 flows down in a liquid phase when the steam sterilization apparatus according to the first exemplary embodiment reoperates is solved by connecting the solenoid valve 110 to the water supply pipe 11.

The solenoid valve 110 operates according to a control signal transferred from the control unit 3 to supply water to the vaporization passage 5 of the heater block 9 or collect the water in the heater block 9 to the water container 1.

A detailed operation of the solenoid valve 110 will be described below.

First, when the steam sterilization apparatus operates in an initial stage, the solenoid valve 110 is opened for a predetermined time by receiving the operation signal from the control unit 3 and the pump 12 operates to collect the water moving along the water supply pipe 11 to the water container 1 through the drain pipe 112 for the predetermined time when the solenoid valve 110 is opened to prevent the water from flowing into the heater block 9 during the heater block 9 is preheated. After the predetermined time elapses, when the heater block 9 is sufficiently heated to vaporize the water that flows in, the solenoid valve 110 is closed to allow the water moving along the water supply pipe 11 to flow into the vaporization passage 5 in the heater block 9.

Second, after the operation of the steam is terminated, the solenoid valve 110 is opened for the predetermined time by receiving the operation signal from the control unit 3 and thereafter, closed like the above case. In this case, the water remaining in the vaporization passage 5 of the heater block 9 is collected to the water container 1 through the drain pipe 112 for the opened time. After the predetermined time elapses, when the water in the heater block 9 is collected to the water container 1 through the drain pipe 112, the solenoid valve 110 is closed to prevent the water from flowing backwards from the water container 1.

The above operation can further reduce the preheating time of the heater block 9 when the steam sterilization apparatus operates and can be further reduced through the above operation and it is possible to prevent the liquid phase water that flows out as it is in the initial operation from deteriorating a sterilization effect.

The heat dissipating pipe 111 is configured by a coil type and as a heat dissipation area is extended, the heat transferred from the heater block 9 is dissipated rapidly to prevent the solenoid valve 110 or the pump 12 from being damaged due to the transfer heat transferred through the water supply pipe 11 made of a metallic material.

Further, a pipe block 130 may be installed in the water supply pipe 11, each of a water pressure balancing unit 140 and a depressurizing unit 150 may be installed in the pipe block 130, and the solenoid valve 110 may also be installed in the pipe block 130.

The water pressure balancing unit 140 is configured to prevent a pulsation phenomenon generated due to the operation of the pump 12 and is configured by a balancing portion 144 mounted on a hole 142 formed in the pipe block 130 and receiving the water supplied to the heater block 9 to remove pulsation.

The balancing unit 144 includes a fixation body 145 fixed to the hole 142 and having upper and lower portions which are opened, a diaphragm 147 placed on the top of the fixation body 145, an elastic body 149 placed on the top of the diaphragm and having a plurality of vertical grooves 143 formed on the top surface thereof to have a plurality of small-sized diaphragms 141 formed on a contact surface with the diaphragm 147, and a cover body 148 protecting the diaphragm 147 and the elastic body 149, and the cover body 148 is fixed and coupled to the fixation body 145 through a bending portion 146 formed on an upper circumference of the fixation body 145. Herein, the diaphragm 147 receives the water that flows into the pipe block 130 to balance the pressure and the elastic body 149 secondarily balances the pressure of the water balanced primarily in the diaphragm 147 to prevent the pulsation phenomenon from occurring in the water moving through the water supply pipe 11, thereby uniformly spraying the steam through the steam nozzle 6.

The depressurizing unit 150 is installed in the pipe block 130 and controls the pressure to match the pressure of the pump 12 to serve to supply the water at the most ideal pressure.

That is, the depressurizing unit 150 includes a depressurization discharge pipe 152 which is in communication with the pipe block 130, a depressurizing valve 154 provided at an outlet of the depressurization discharge pipe 152 and setting a depressurization degree of water pressure, and a water collecting pipe 156 interconnecting the depressurizing valve 154 and the water container 1.

Hereinafter, an operational relationship of the steam sterilization apparatus according to the second exemplary embodiment of the present invention configured as above will be described.

In the case where sterilization is performed by using the steam sterilization apparatus according to the second exemplary embodiment of the present invention, the apparatus is first operated by user's operation, the control unit 3 increases the temperature of the heater plate 42 by applying power to the heater block 9 according to an operation signal, operates the pump 12 installed in the water supply pipe 11, and opens the solenoid valve 110 for a predetermined time and thereafter, closes the solenoid valve 110.

Therefore, for a time (approximately 7 seconds) when the solenoid valve 110 is opened, air and water filled in the pipe block 130 flows into the drain pipe 112 through the solenoid valve 110 to be collected to the water container 1 and the body 90 of the heater block 9 is preheated to vaporize the water rapidly.

Thereafter, when the solenoid valve 110 is closed, the water that flows into the pipe bock 130 passes through the water supply pipe 11 and the heat dissipating pipe 111 by the pressure of the pump 12 to flow into the vaporization passage 5 in the preheated body 90 and rapidly vaporized in the preheated vaporization passage 5 to be sprayed to the outside through the steam nozzle 6 by its own pressure.

By this method, a time when the steam is first sprayed from the steam sterilization apparatus can be reduced.

Next, vacuum pressure is applied to the upper portion of the liquid chemical nozzle 21 placed close in a direction perpendicular to the front portion of the steam nozzle 6 while the steam is sprayed through the steam nozzle 6 and the liquid chemical stored in the liquid chemical container 2 is discharged through the liquid chemical nozzle 21 via the liquid chemical supply pipe 22 due to the vacuum pressure applied to the upper portion of the liquid chemical nozzle 21 and the discharged liquid chemical is mixed with the steam sprayed through the steam nozzle 6 to be sprayed to the outside.

Meanwhile, after the operation of the steam sterilization apparatus is terminated, the pump 12 and the heater block stop according to the operation signal of the control unit 3 and the solenoid valve 110 is opened for a predetermined time and thereafter closed.

In this case, for the time when the solenoid valve 110 is opened, the pressure of the water remaining in the vaporization passage 5 is increased due to remaining heat of the heater block 9 and the water with the increased pressure flows backwards to pass through the solenoid valve 110 and thereafter, flows into the water container 1 through the drain pipe 112. Thereafter, when the water remaining in the vaporization passage 5 is collected to the water container 1, the solenoid valve 110 is closed to prevent the water stored in the water container 1 from flowing backwards through the drain pipe 112 again.

By this method, it is possible to prevent the liquid phase water from being sprayed as it is in advance when the steam sterilization apparatus reoperates.

### [Industrial Applicability]

A steam sterilization apparatus according to the present invention is configured to mix different fluids and spray the mixed fluids to be used in service industries such as cleaning or sterilization or a painting industry.

## Claims

1. A steam sterilization apparatus, comprising:
a case forming a main body, a water container arranged in an inner rear portion of the case to store water;
a liquid chemical container arranged behind the water container to store a liquid chemical;
a nozzle assembly which is arranged in an inner front portion of the case and which includes a steam nozzle for receiving water from the water container and spraying water to the outside and a liquid chemical nozzle for receiving a liquid chemical from the liquid chemical container and discharging the liquid chemical to the outside;
a water supply pipe which interconnects the water container and the steam nozzle;
a liquid chemical supply pipe which interconnects the liquid chemical container and the liquid chemical nozzle;
a heater block interposed between the water supply pipe and the steam nozzle to heat and vaporize the water supplied through the water supply pipe;
a pump installed at the water supply pipe to apply pressure to the water in the water container and to supply water to the heater block through the water supply pipe; and
a control unit which controls the operation of the pump and of the heater block.

2. The steam sterilization apparatus of claim 1, wherein the steam nozzle and the liquid chemical nozzle constituting the nozzle assembly are placed close to each other in a direction perpendicular to each other.

3. The steam sterilization apparatus of claim 1 or 2, wherein:
a solenoid valve is additionally provided in the water supply pipe, and
the solenoid valve is connected with the water container through a drain pipe.

4. The steam sterilization apparatus of claim 3, wherein:
The solenoid valve is opened for a predetermined time to preheat the heater block when the pump and the heater block operate in the initial stage to collect the water supplied through the water supply pipe to the water container through the drain pipe and is closed after the preheating is completed, and is opened for a predetermined time again after the operation of the pump and the heater block is terminated to collect water remaining the heater block to the water container through the drain pipe.

5. The steam sterilization apparatus of claim 1 or 2, wherein the heater block includes:
a main body with a vaporization passage vaporizing the water that flows thereinto while moving the water to an upper portion thereof; and
a pair of heater plates provided at both sides of the body, respectively and heating the body to vaporize the water in the vaporization passage.

6. The steam sterilization apparatus of claim 5, wherein the vaporization passage includes multi-stage horizontal passages and a plurality of vertical communication holes interconnecting the multi-stage horizontal passages.

7. The steam sterilization apparatus of claim 6, wherein in the multi-stage horizontal passages, a center shaft is provided with a predetermined distance from an inner periphery of the horizontal passage to form a vaporization space between the inner periphery of a horizontal path and an outer periphery of the center shaft.

8. The steam sterilization apparatus of claim 7, wherein a plurality of protruded portions and buried portions are formed on the inner periphery of the horizontal passage and the outer periphery of the center shaft to increase a vaporization area in a longitudinal direction.

9. The steam sterilization apparatus of claim 6, wherein a steam temperature sensor for measuring the temperature of steam passing through the vertical communication hole is further provided in the plurality of vertical communication holes.

10. The steam sterilization apparatus of claim 5, wherein a pair of spring plates for fixing the pair of heater plates to the heater block, respectively are further provided in the heater block.

11. The steam sterilization apparatus of claim 1 or 2, wherein a block temperature sensor for measuring the temperature of the heater block is further provided in the heater block.

12. The steam sterilization apparatus of claim 11, wherein horizontal fixing bars for fixing the heater block to the case are provided at both sides of the block temperature sensor in the heater block.

13. The steam sterilization apparatus of claim 1 or 2, wherein a heat dissipating pipe for preventing heat generated from the heater block from being transferred to the water supply pipe is further provided at a connection portion between the water supply pipe and the heater block.

14. The steam sterilization apparatus of claim 1 or 2, wherein:
a pipe block is installed in the water supply pipe, and
a water pressure balancing unit for preventing pulsation of supplied water is further provided in the pipe block.

15. The steam sterilization apparatus of claim 14, wherein the water pressure balancing unit includes:
a diaphragm primarily balancing the pressure by receiving the water that flows into the pipe block; and
an elastic body closely attached to the top of the diaphragm to secondarily balance the pressure of the water.

16. The steam sterilization apparatus of claim 15, wherein a plurality of vertical grooves in which upper portions are opened and a small-sized diaphragm is formed in each of lower portions are further provided in the elastic body.

17. The steam sterilization apparatus of claim 14, wherein a depressurizing unit adjusting the water pressure is further provided in the pipe block.

18. The steam sterilization apparatus of claim 17, wherein the depressurizing unit includes:
a depressurization discharge pipe which is in communication with the pipe block;
a depressurizing valve provided at an outlet of the depressurization discharge pipe and setting a depressurization degree of the water pressure; and
a water collecting pipe interconnecting the depressurizing valve and the water container.

19. The steam sterilization apparatus of claim 1 or 2, wherein:
a liquid chemical container receiving unit for mounting the liquid chemical container to be attachable or detachable is formed in an inner rear portion of the case, and
a cable mounting space on which a power cable is mounted is formed in a lower portion of the liquid chemical container receiving unit with being a lower portion thereof opened.

20. The steam sterilization apparatus of claim 19, wherein an upper cover unit which is configured to be opened through a front hinge portion and expose the liquid chemical container to the outside of the case when being opened is formed on a rear top of the case.

21. The steam sterilization apparatus of claim 19, wherein:
a buried groove having a predetermined depth is formed on a bottom of the liquid chemical container, and
a protruded portion for inserting and coupling to the buried groove is formed in the liquid chemical container receiving unit.

22. The steam sterilization apparatus of claim 19, wherein:
a protruded pipe opened eccentrically in a direction in which the liquid chemical container is mounted is formed on the top of the water container and a helix portion is formed in an outer diameter of the protruded pipe, and
a press plug joined to the helix portion of the protruded pipe to close the protruded pipe 31 is coupled to the protruded pipe, however, one portion of the press plug joined to the helix portion presses an upper portion of the liquid chemical container mounted on the liquid chemical container receiving unit to prevent the liquid chemical container mounted on the liquid chemical container receiving unit from being removed
